Europäisches Patentamt

European Patent Office

Office européen des brevets

(11) Veröffentlichungsnummer: **0 240 560 B1**

(12) **EUROPÄISCHE PATENTSCHRIFT**

(45) Veröffentlichungstag der Patentschrift: **03.06.92**

(51) Int. Cl.5: **C12M 3/00**, C12M 1/06, C12M 1/12

(21) Anmeldenummer: **86906301.6**

(22) Anmeldetag: **02.10.86**

(86) Internationale Anmeldenummer:
**PCT/EP86/00566**

(87) Internationale Veröffentlichungsnummer:
**WO 87/02054 (09.04.87 87/08)**

(54) **VORRICHTUNG UND VERFAHREN ZUR BLASENFREIEN BEGASUNG VON FLÜSSIGKEITEN, INSBESONDERE VON KULTURMEDIEN ZUR VERMEHRUNG VON GEWEBEKULTUREN.**

(30) Priorität: **02.10.85 DE 3535183**

(43) Veröffentlichungstag der Anmeldung:
**14.10.87 Patentblatt 87/42**

(45) Bekanntmachung des Hinweises auf die
Patenterteilung:
**03.06.92 Patentblatt 92/23**

(84) Benannte Vertragsstaaten:
**AT BE CH DE FR GB IT LI NL SE**

(56) Entgegenhaltungen:
**EP-A- 0 172 482**
**WO-A-85/02195**
**GB-A- 2 059 436**
**GB-A- 2 093 730**

(73) Patentinhaber: **Gesellschaft für Biotechnologische Forschung mbH (GBF)**
**Mascheroder Weg 1**
**W-3300 Braunschweig-Stöckheim(DE)**

(72) Erfinder: **BÜNTEMEYER, Heino**
**Im Schapenkamp 26**
**W-3300 Braunschweig(DE)**
Erfinder: **LEHMANN, Jürgen**
**Bolkenhain 2 d**
**W-3300 Braunschweig(DE)**

(74) Vertreter: **Boeters, Hans Dietrich, Dr. et al**
**Boeters & Bauer Bereiteranger 15**
**W-8000 München 90(DE)**

EP 0 240 560 B1

## Beschreibung

Vorrichtungen zur blasenfreien Begasung von Flüssigkeiten, insbesondere von Kulturmedien zur Vermehrung von Gewebekulturen, werden in Reaktoren eingesetzt, die neben dem Gas-Versorgungs/Entsorgungs-System regelmäßig ein System zum zellfreien Medienwechsel umfassen. Beispielsweise ist bei der Kultivierung tierischer Zellen in Bioreaktoren der zellfreie Medienwechsel problematisch, wenn Zelldichten von mehr als $1 \times 10^7$ Zellen/ml erreicht werden sollen.

Für den kontinuierlichen zellfreien Medienwechsel sind beispielsweise auf einer Rührerwelle angebrachte Spinnfilter vorgeschlagen worden; Develop. biol. Standard, 60 (1985) 229 bis 236. Hinsichtlich der Nachteile, die sich beim Rühren von Gewebekulturen ergeben, kann auf die DE-A- 3 430 924 verwiesen werden. Ferner sind externe Kreisläufe mit Filtereinheiten und/oder Sedimentationsstrecken für Mikrocarrierkulturen vorgeschlagen worden; Ann. Rep. Ferment. Proc., 6 (1983) 35 bis 74. Bei externen Kreisläufen treten jedoch hohe Scherkräfte durch das kontinuierliche Umpumpen der Zellsuspensionen auf, so daß die Zellen stark geschädigt werden. Flachmembranen sind verstopfungsanfällig und befriedigen auch hinsichtlich des Flüssigkeitsdurchsatzes wenig.

Aus EP-A-0 172 478 = 85.109 767.5 sind eine Vorrichtung und ein Verfahren zur blasenfreien Begasung von Flüssigkeiten, insbesondere von Kulturmedien zur Vermehrung von Gewebekulturen, mit einem Gasaustausch über eine eingetauchte Membranfläche, die mit Hilfe eines Membrankorbes vorgesehen ist, der pendelnd bewegt wird und einen oder mehrere hydrophobe poröse Polypropylen-Membranhohlfäden aufweist, bekannt. Es bestand jedoch ein Bedürfnis, diesen Stand der Technik noch zu verbessern.

Dazu wird erfindungsgemäß eine Vorrichtung gemäß dem Oberbegriff von Anspruch 1 vorgeschlagen, die dadurch gekennzeichnet ist, daß der Membrankorb zusätzlich mit einem oder mehreren hydrophilen porösen Membranhohlfäden versehen ist, die durch Hydrophilisieren poröser Polypropylen-Membranhohlfäden durch Benetzen mit einem organischen Lösungsmittel und anschließendes Benetzen mit Wasser erhältlich sind.

Diese hydrophilen porösen Membranhohlfäden bilden zusammen mit den hydrophoben porösen Membranhohlfäden den Membrankorb.

Eine bevorzugte erfindungsgemäße Vorrichtung ist durch mehrere hydrophobe poröse Polypropylen-Membranhohlfäden und mehrere hydrophile poröse Polypropylen-Membranhohlfäden gekennzeichnet, die jeweils getrennt beströmbar sind.

Erfindungsgemäß wird ferner ein Verfahren zur blasenfreien Begasung von Flüssigkeiten, insbesondere von Kulturmedien zur Vermehrung von Gewebekulturen vorgesehen, das dadurch gekennzeichnet ist, daß man das Verfahren mit einer erfindungsgemäßen Vorrichtung durchführt. Für das erfindungsgemäße Verfahren ist es vorteilhaft, wenn man intermittierend über die hydrophilen porösen Polypropylen-Membranhohlfäden flüssiges Medium in den Reaktor einleitet und flüssiges Medium aus dem Reaktor abzieht.

Unter der Pendelbewegung des Membrankorbes werden eine translatorische Bewegung, eine lineare Pendelbewegung, eine Kreispendelbewegung, eine Kreisschwingbewegung und Kombinationen dieser Bewegungen verstanden. Eine einsinnige Drehbewegung um eine Rotationsachse (ohne Bewegungsumkehr) ist wegen der damit verbundenen Scherwirkung nicht vorteilhaft.

Bei den erfindungsgemäß vorgesehenen hydrophoben porösen bzw. mikroporösen Membranhohlfäden kommt es zur Begasung über die Membranporen. Die Membranporen sind mit Gas gefüllt, so daß die Gas/Flüssigkeits-Phasengrenze etwa in den Poren etwa in der Ebene der Außenfläche der Membranhohlfäden liegt und flüssiges Medium nicht in die Membranhohlfäden eintritt. Die Lage der Phasengrenze kann dabei durch geeignete Wahl des Drucks in den Membranhohlfäden gegen den herrschenden Außendruck eingestellt werden.

Die Verwendung von Membranhohlfäden aus Polypropylen hat sich als vorteilhaft erwiesen, beispielsweise aus Polypropylen gemäß DE-A-31 07 874. Poröse bzw. mikroporöse Membranhohlfäden aus Polypropylen sind per se hydrophob. Man kann diese Membranhohlfäden jedoch dadurch hydrophilisieren, daß man sie mit einem organischen Lösungsmittel und anschließend mit Wasser benetzt. Diese Hydrophilisierung läßt sich durch Trocknen rückgängig machen. Der Membrankorb kann also aus Polypropylen-Membranhohlfäden gebildet werden, die einerseits unbehandelt, andererseit in bequemer Weise hydrophilisiert worden sind.

Wenn man abwechselnd über die hydrophilen Membranhohlfäden flüssiges Medium einleitet und flüssiges Medium aus dem Reaktor abzieht, erreicht man eine vorteilhafte Spülung der Membranhohlfäden.

Nachstehend wird die Erfindung durch sechs Figuren und vier Beispiele näher erläutert.

Figuren 1 bis 3 zeigen drei Fermentationen gemäß den Beispielen 1 bis 3;

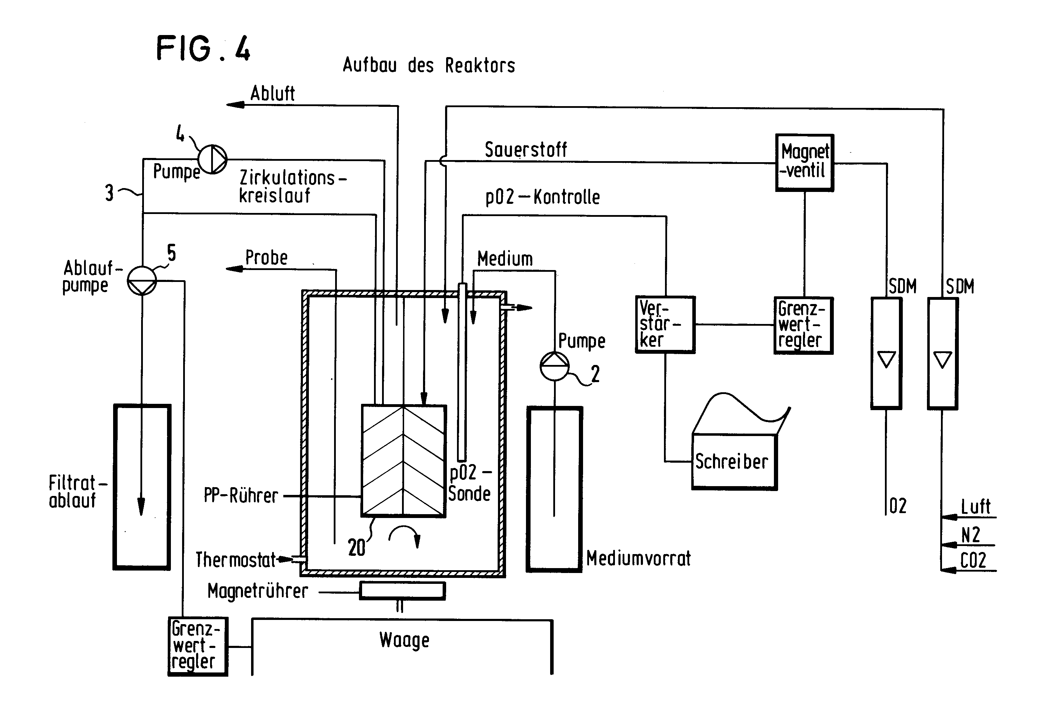

Figur 4 zeigt schematisch die in den Beispielen 1 bis 3 verwendete Vorrichtung;

Figuren 5 bis 6 zeigen Reaktoren mit erfindungsgemäßen Membrankörben mit hydrophoben porösen Membranhohlfäden.

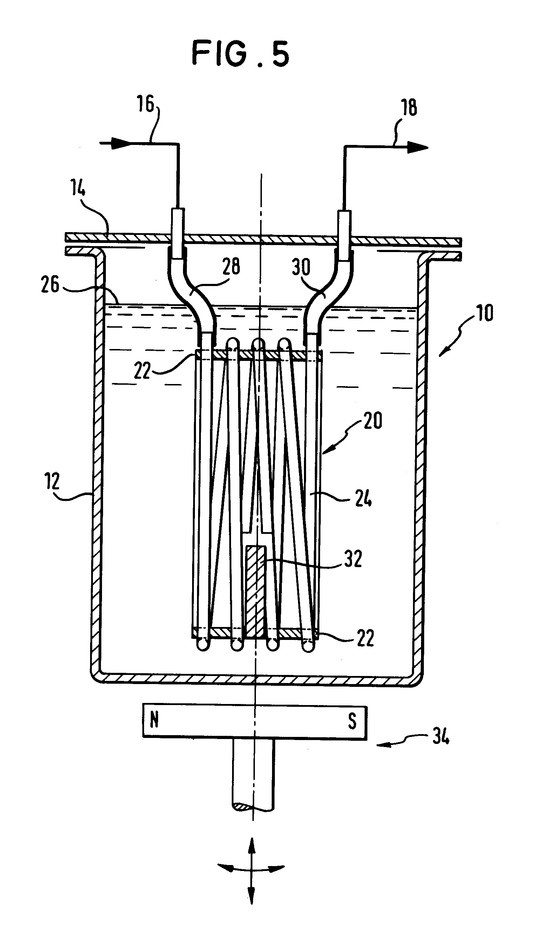

Der in der Figur 5 dargestellte schematische Aufbau einer erfindungsgemäßen Vorrichtung 10 besteht aus einem unmagnetischen Behälter 12, auf dem ein Deckel 14 angeordnet ist, der gegebenenfalls in nicht dargestellter Weise befestigt und abgedichtet sein kann. Durch den Deckel 14 sind eine Gaseintrittsleitung 16 und eine Gasaustrittsleitung 18 geführt.

In dem Behälter 12 ist ein Membrankorb 20 angeordnet, der einen hohlzylinderförmigen Membranträger 22 besitzt, auf den Membranhohlfäden 24 schraubenartig aufgewickelt sind. Der Membrankorb 20 ist in eine sich in dem Behälter 12 befindende Flüssigkeit 26 vollständig eingetaucht und wird an seinem oberen Ende von elastischen Schläuchen 28 und 30 flexibel gehalten. Der Schlauch 28 ist mit der.

Gaseintrittsleitung 16 und einem Ende des aufgewickelten Membranhohlfadens 24 verbunden, während der Schlauch 30 das andere Ende des aufgewickelten Membranhohlfadens 24 mit der Gasaustrittsleitung 18 verbindet.

Im unteren Bereich des Membrankorbs 20 ist an dem Membranträger 22 drehfest ein Magnetkern 32 angebracht, der von einer Magnetanordnung 34 beaufschlagbar ist, die außerhalb des Behälters 12 angeordnet ist. Die Magnetanordnung 34 ist über eine nicht dargestellte Einrichtung linear hin- und her beweg bar, um über den Magnetkern 32 dem Membrankorb 20 eine Pendel bewegung zu vermitteln. Diese lineare Bewegung erfolgt in einer derartigen Richtung, daß der Membrankorb 20 um eine durch die Aufhängungspunkte laufende Gerade pendelt.

Im unteren Bereich des Membrankorbs 20 ist an dem Membranträger 22 drehfest ein Magnetkern 32 angebracht, der von einer Magnetanordnung 34 beaufschlagbar ist, die außerhalb des Behälters 12 angeordnet ist. Die Magnetanordnung 34 ist über eine nicht dargestellte Einrichtung drehbar, um über den Magnetkern 32 dem Membrankorb 20 eine Kreispendelbewegung zu vermitteln. Bei dieser Kreispendelbewegung bewegt sich der Membrankorb ohne eigene Drehung um seine Symmetrieachse auf einer Kreisbahn.

Alternativ ist es auch möglich, eine lineare Bewegung unter einem bestimmten Winkel zur Symmetrieachse des Membrankorbs einzuleiten, um einer linearen Pendelbewegung des Membrankorbs aufgrund der Elastizität der Schläuche 28 und 30 eine zusätzliche Bewegungskomponente zu verleihen, die zu einer elliptischen bzw. einer kreisförmigen Pendelbewegung führen kann. Es ist darüber hinaus auch möglich, anstelle der entsprechend bewegten Magnetanordnung 34 eine stationäre Elektromagnetanordnung vorzusehen, wobei die gewünschte Pendel- oder Drehpendelbewegung des Membrankorbs durch eine entsprechende Anordnung und Ansteuerung der Elektromagnete erreicht werden kann.

Die Begasungsrate läßt sich durch Änderung der Kreispendelfrequenz und/oder des Radius der Kreisbahn steuern. Bei dem dargestellten Ausführungsbeispiel kann eine entsprechende Änderung des Kreisbahnradius in einfacher Weise dadurch vorgenommen werden, daß in dem Deckel 14 der Abstand zwischen der Gaseintrittsleitung 16 und der Gasaustrittsleitung 18 verändert wird. Beispielsweise kann durch Verkleinerung des genannten Abstandes die Membrankorbauslenkung aus der Ruhelage (d.h. derjenigen Lage, in der dem Membrankorb 20 keine Bewegung aufgeprägt wird und dieser unbewegt in dem Behälter 12 hängt) entsprechend verringert werden.

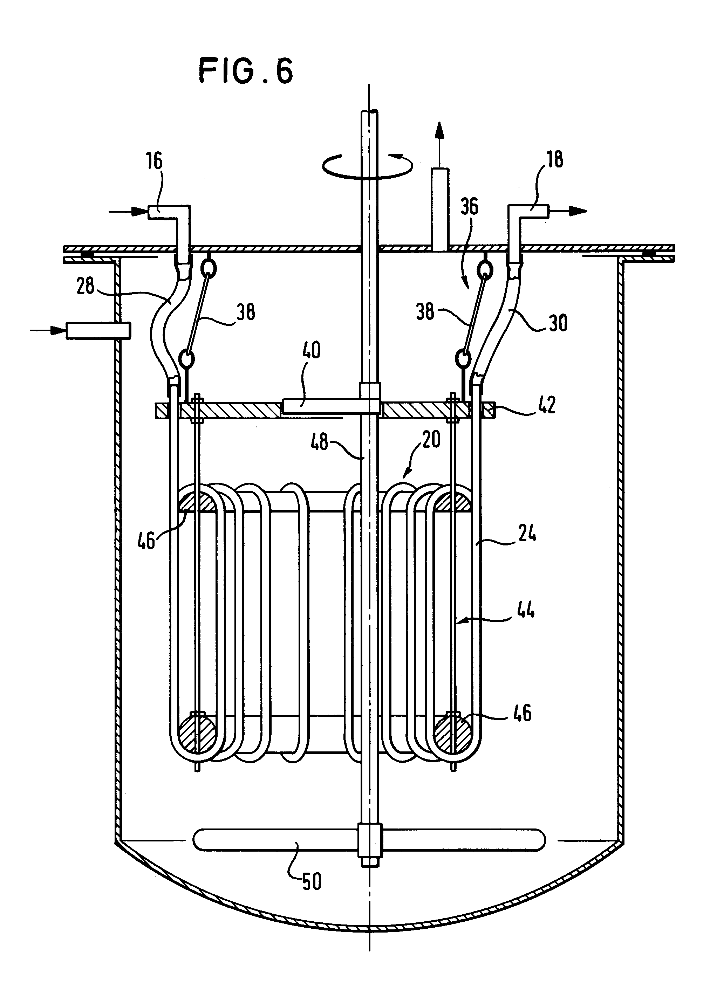

Das in der Figur 5 dargestellte Ausführungsbeispiel ist vor allem für kleinere Flüssigkeitsmengen vorgesehen. Für die blasenfreie Begasung von Flüssigkeitsmengen in der Größenordnung von 30 l und mehr wird gemäß Figur 6 die Aufhängung über die elastischen Schläuche 16, 18 für die Zu- und Abfuhr von Gas vorteilhaft durch eine geeignete Aufhängung 36 mit biegsamen oder schwenkbaren Gelenkgliedern (38) vorgenommen und der maßstäblich vergrößerte Membrankorb 20 mit Hilfe eines Exzenters 40 bewegt. Die Gelenkglieder 38 können beispielsweise aus zwei oder mehr gleichlangen Zugdrähten bestehen, die mit Abstand voneinander an dem Deckel 14 gelenkig angreifen und gelenkig mit einer den Exzenter 40 umgebenden Führungsscheibe 42 verbunden sind, deren Symmetrieachse mit der des Membrankorbs 20 zusammenfällt. Diese Führungsscheibe 42 kann mittels eines Spannringträgers 44 an geschlitzten Spannringen 46 befestigt sein, die mit dem Membranhohlfaden oder den Membranhohlfäden 24 den entsprechend vergrößerten Membrankorb bilden. Zur Aufprägung einer Kreispendelbewegung auf den Membrankorb 20 kann eine in den Behälter 12 eingreifende Antriebswelle 48 vorgesehen sein, die mit dem in seiner Exzentrizität einstellbaren Exzenter 40 an der bzw. in die Führungsscheibe 42 eingreift. Die Antriebswelle 48 tritt dabei vorzugsweise durch den Deckel 14 oder den Boden des Behälters 12 im Bereich der Behältersymmetrieachse ein. Falls die Antriebswelle 48 durch den Deckel 14 des Behälters 12 eingeführt wird, ist die Führungsscheibe 42 vorteilhaft oberhalb des Membrankorbs 20 angeordnet, und die Welle 48 kann durch den hohlzylindrischen Membrankorb 20 hinaus in die untere Hälfte des Behälters 12 verlängert und an ihrem unteren Ende mit einem Rührer 50 versehen sein. Dieser Rührer 50 kann während der ersten Züchtungsstufe einer Kultur in der sogenannten Anzuchtsphase bei einem niedrigen Flüssigkeitspegel und reiner Belüftung über die Oberfläche eine langsame Bewegung der Flüssigkeit vornehmen.

Die zuvor beschriebene Vorrichtung ist auch als Zusatzsystem für herkömmliche Fermenter geeignet und bildet eine ideale Ergänzung für vorhandene Systeme.

Beispiel 1

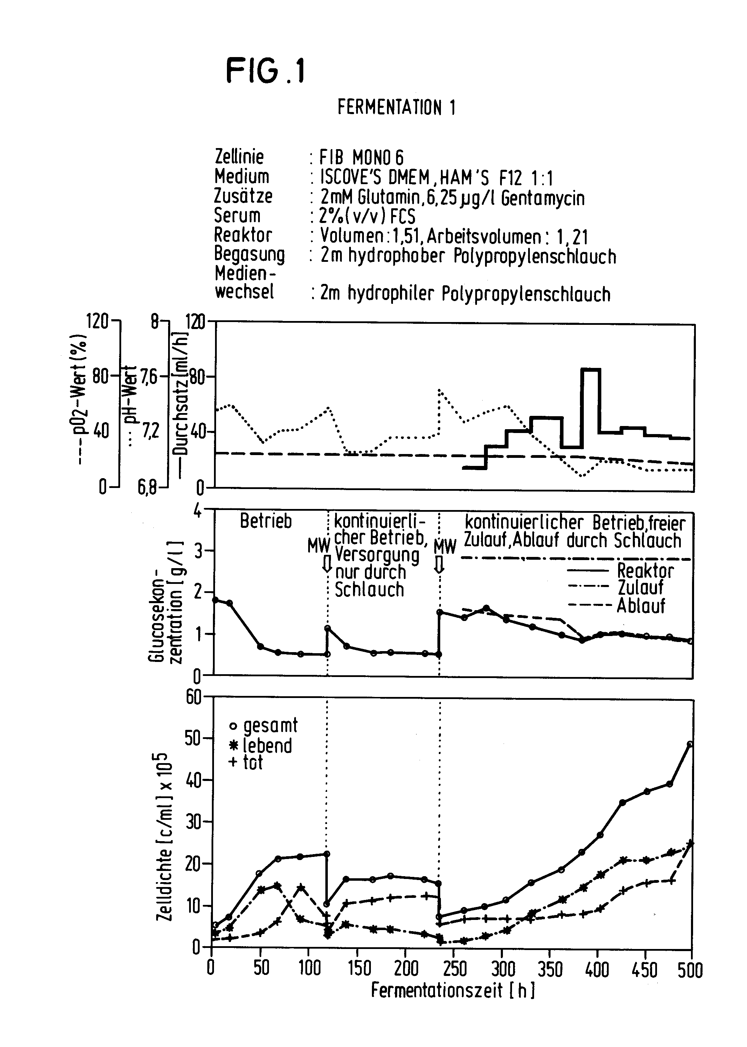

Zur Untersuchung der Leistungsfähigkeit der in der Figur 1 schematisch dargestellten Vorrichtung wurde ein Membrankorb 20 hergestellt, auf dem ein hydrophober Membranhohlfaden 24 aus Accurel$^R$ Typ PX 190/1/2/8366 mit einem Porenvolumen von 75 % und einer Porengröße von 0,3 $\mu$m aufgewickelt worden war. Die Länge des Membranhohlfadens 24 betrug 121 cm, die äußere Membranoberfläche 98,7 cm², und der Membranhohlfaden wurde mit reinem Sauerstoff bei einem Überdruck von 2,9 mbar (30 mm WS) von innen begast. Als Membranmaterial ist besonders mikroporöses Polypropylen, beispielsweise Accurel$^R$, günstig.

Der Membrankorb wurde in 1,3 l destilliertes Wasser eingetaucht und mit einer Auslenkung von 2,5 cm um die Mitte mit 60 Kreispendelungen pro Minute durch einen unter dem Behälter drehbar angeordneten Magneten bewegt.

Messungen an dieser Vorrichtung ergaben, daß bei einer Temperatur von 25 °C eine Sauerstoffübertragungsrate OTR von 56 mg O₂/ (1·h) ohne Austreten von Sauerstoffblasen aus der Membranoberfläche erreicht werden konnte. Dies entspricht einem spezifischen Sauerstofffluß von

$$Q_{O_2}$$

von 0,57 mg O₂/ (cm²·h). An der unbewegten Oberfläche wurde an der gleichen Vorrichtung ein Sauerstofffluß

$$Q_{O_2}$$

von 0,22 mg O₂/(cm²·h) gemessen.

Die beschriebene Vorrichtung ist beispielsweise für die Züchtung von Fibroblasten, z. B. der humanen diploiden Vorhautzellen (FS-4), geeignet, die einen spezifischen Sauerstoffbedarf

$$q_{O_2}$$

von 0,05 mmol O₂/(10⁹ Zellen·h) oder 1,6 mg O₂/(10⁹ Zellen·h) haben. Es wurde berechnet, daß mit dieser Vorrichtung 3,5 · 10¹⁰ Zellen/l versorgt werden könnten.

Nachfolgend wird die Anwendung des Verfahrens und der Vorrichtung in einem anderen Beispiel beschrieben. Eine Zellkultur mit primären menschlichen Leukozyten mit einer Zellzahl n von 4,5 · 10⁹ Zellen/l und einem anfänglichen spezifischen Sauerstoffbedarf

$$q_{O_2}$$

von 4,3 mg O₂/(10⁹ Zellen . h) wurde bei 37 °C in einem Kulturmedium RPMI 1640 und einigen prozeßbedingten Zusätzen bei 60 Kreispendelungen pro Minute und einem Sauerstoffüberdruck von 2,9 mbar (30 mm WS) behandelt. Dabei wurde eine Sauerstoffübertragungsrate OTR von 38 mg O₂/l.h) bei blasenfreier Begasung erreicht. Dieser Wert übersteigt den notwendigen Wert um 18,5 mg O₂/-(l.h). Um die Sauerstoffübertragungsrate auf den Bedarf von 19,5 mg O₂/(l.h) zu verringern, wurde eine einfache Absenkung der Kreispendelfrequenz vorgenommen.

Eine bedarfsabhängige Steuerung der Sauerstoffversorgung kann auch durch eine Veränderung der Gaskonzentration im einströmenden Gas erreicht werden. Bei der vorstehend beschriebenen Vorrichtung wurde beispielsweise eine Regelung vorgenommen, bei der ein Sollwert von 10 % Luftsättigung derart aufrecht erhalten wurde, das durch die Membran ein kontinuierlicher Luftstrom von 1 Nl/h geleitet wurde, dem im Bedarfsfall reiner Sauerstoff zugemischt wurde. Die Zumischung erfolgte mittels eines durch einen Zweipunkt-Regler gesteuerten Magnetventils, wobei der Istwert der Luftsättigung im Medium mit einer Sauerstoffelektrode gemessen wurde.

Die in der Zellkulturtechnik übliche Einstellung des pH-Wertes über ein Puffer-CO₂-Gleichgewicht läßt sich durch Zumischen der notwendigen CO₂-Menge in das Eintrittsgas erreichen.

Das erfindungsgemäße Verfahren und die erfindungsgemäße Vorrichtung eignen sich auch in vorteilhafter Weise für ein blasenfreies Begasen von Mikrocarrier-Kulturen.

Beispiele 2 bis 4

Die für die hydrophoben und hydrophilen Membranhohlfäden gewählten Schläuche bestandem aus mikroporösem, hydrophobem Polypropylen (Accurel$^R$, ENKA AG) und hatten folgende Daten: $D_i$ = 1.88 mm, WS = 0.4 mm, Porengröße 0.3 $\mu$m, Außenoberfläche 81.7 cm²/m. Zur Hydrophilisierung wurde einer der Schläuche 1 h lang mit 70-proz. Ethanol behandelt und danach sorgfältig mit reinem Wasser (Milli-Q) bis zur Alkoholfreiheit gespült.

Die beiden Schläuche wurden im 1.5 -Liter-Maßstab bei drei Fermentationen eines Maus-Maus-Hybridoms eingesetzt. Dazu wurde zuerst die hydrophobe Schlauchwicklung gemäß Figur 5 und danach (in feuchtem Zustand) die hydrophile Schlauchwicklung auf einen magnetisch angetriebenen Pendelrührer 20 gemäß Figur 5 aufgebracht. Die jeweiligen Schlauchlängen für Begasung und Medienwechsel sind den Figuren 1 bis 3 zu entnehmen. Wie die Ergebnisse zeigen, ist dabei ein Verhältnis der Schlauchlängen von Begasungschlauch und Medienwechselschlauch von 3-4 m/l zu 1 m/l für Zelldichten um $2 \times 10^7$ Zellen/ml zweckmäßig. Weder der hydrophobe noch der hydrophile Schlauch veränderten ihre Eigenschaften während der Dampfsterilisation.

Das kontinuierliche Verfahren lief wie folgt ab: Der Zulauf an frischem Medium (Nährstoffe) erfolgte frei über eine Dosierpumpe 2. Zum Abpumpen von zellfreiem Medium durch den hydrophilen Schlauch wurde im Hinblick auf eine mögliche Verstopfung der Poren und im Hinblick auf die Flexibilität des Schlauches ein Zirkulationskreislauf 3 vorgesehen. Mit Hilfe einer externen Pumpe 4 wurde Medium durch den hydrophilen Schlauch zirkuliert. Mit einer weiteren Pumpe 5 konnte dann aus diesem Kreislauf 3 die entsprechende Ablaufmenge entnommen werden. Dabei betrug der Ablauffluß nicht mehr als die Hälfte des Zirkulationsflusses, um das Verhältnis von Porenströmung zur Schlauchinnenströmung nicht zu groß werden zu lassen. Messungen zeigten, daß durch 1 m hydrophilen Schlauch mindestens 150 ml Flüssigkeit/h entfernt werden konnten ($\hat{=}$ dreifaches Arbeitsvolumen/d). Die Fermentationsdauer betrug 135 h, 240 h bzw. 500 h bei unterschiedlich hohen Zelldichten. Während dieser Zeiten konnte keine Beeinträchtigung der Leistungsfähigkeit des hydrophilen Schlauches festgestellt werden, wodurch keine Rückspülungen und Regenerationsphasen erforderlich wurden.

Die Ergebnisse der drei Fermentationen sind in den Figuren 1 bis 3 dargestellt.

Abbildung 1 zeigt das Verhalten einer Batchkultur (Beispiel 2) mit anschließender kontinuierlicher Kultur mit Zellrückhaltung. Die maximale Lebendzelldichte der Batchkultur betrug ca. $1.5 \times 10^6$ Zellen/ml, einen üblichen Wert auch anderer Fermentationen. Mit Hilfe der Zellrückhaltung ließ sich bei diesem Beispiel eine Lebendzelldichte von ca. $2.5 \times 10^6$ Zellen/ml erreichen. Durch den hohen Anteil an toten, lysierten Zellen und deren störender Effekt zu Beginn des kontinuierlichen Teils ($t_F$ = 235 h) konnte eine noch höhere Zelldichte nicht erreicht werden.

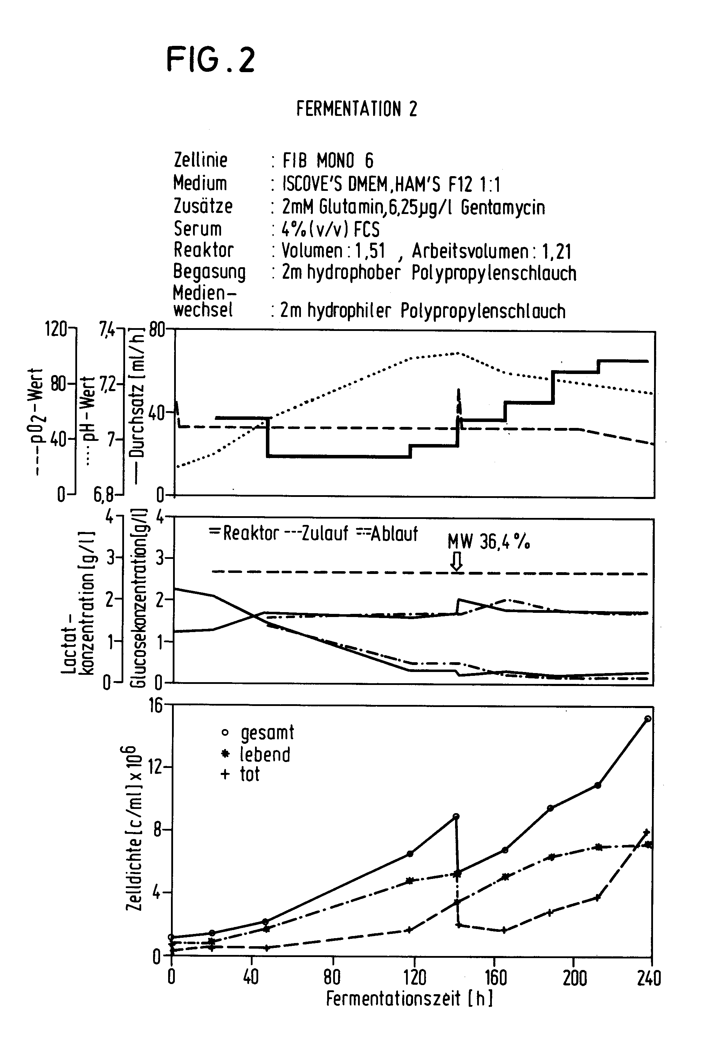

Abbildung 2 zeigt die Ergebnisse einer zweiten Fermentation (Beispiel 3) mit der gleichen Zellinie. Bei diesem Versuch wurde mit einer hohen Zelldichte (Lebendzelldichte $8 \times 10^5$ Zellen/ml) angeimpft und nach 24 h mit dem kontinuierlichen Prozeß begonnen. Innerhalb von 240 h konnte eine Lebendzelldichte von $7.2 \times 10^6$ Zellen/ml trotz eines einmaligen Entfernens von 36 % der Zellmasse erreicht werden. Dabei wurde die Sauerstoffversorgung der Zellen zunehmend problematischer.

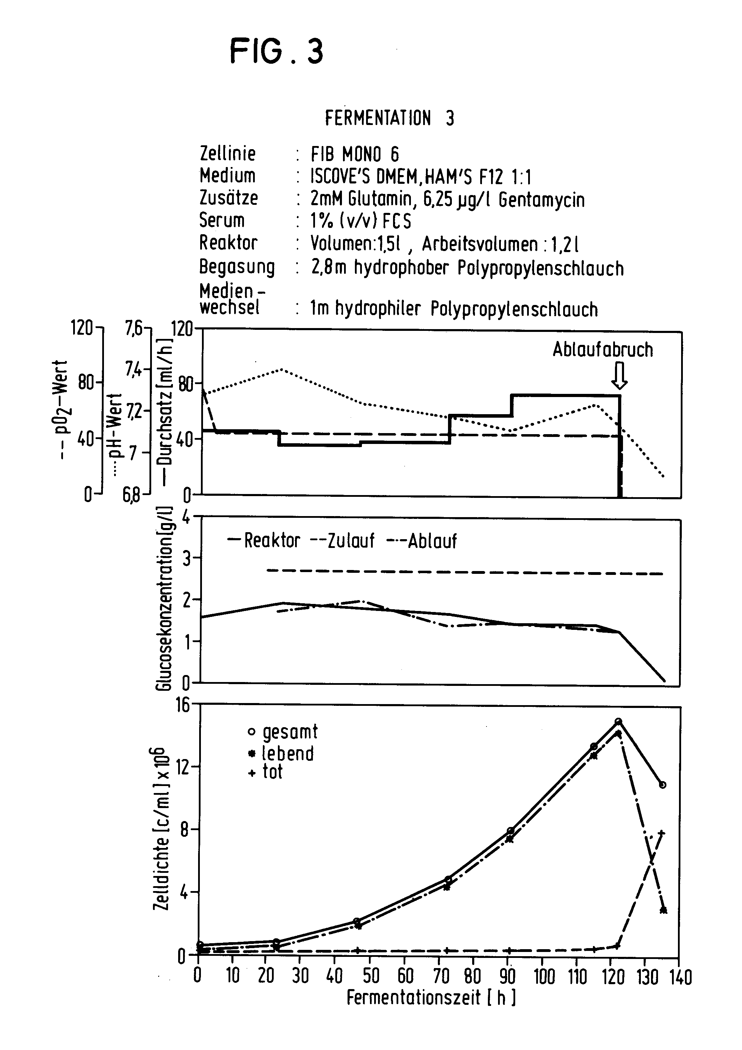

In Abbildung 3 sind die Ergebnisse der dritten Fermentation (Beispiel 4) mit der gleichen Zellinie dargestellt. Diesmal wurde mit niedriger Zelldichte angeimpft (Lebendzelldichte $3.5 \times 10^5$), aber sofort der kontinuierliche Prozeß gestartet. Schon nach 120 h war eine Lebendzelldichte von $1.5 \times 10^7$ Zellen/ml erreicht. Bei dieser Zelldichte konnte die Versorgung mit Sauerstoff (2.8 m Schlauch) noch gewährleistet werden.

Die Ergebnisse der drei Fermentationen demonstrieren deutlich, daß mindestens 100 ml Medium pro Meter Schlauch und Stunde aus dem Reaktor entfernt werden konnten, was der Versorgung von ca. $2 \times 10^7$ Zellen/ml entsprach. Dabei konnten keine Probleme bezüglich der Leistungsfähigkeit des Schlauches festgestellt werden (Verstopfung, Kollabieren).

| | |
|---|---|
| 2 | Dosierpumpe |
| 3 | Zirkulationskreislauf |
| 4 | externe Pumpe |
| 5 | Pumpe |
| 10 | Vorrichtung |
| 12 | Behälter |
| 14 | Deckel |
| 16 | Gaszutrittsleitung |
| 18 | Gasaustrittsleitung |
| 20 | Membrankorb |
| 22 | Membranträger |
| 24 | Membranhohlfaden |
| 26 | Flüssigkeit |
| 28 | Schlauch |
| 30 | Schlauch |
| 32 | Magnetkern |
| 34 | Magnetanordnung |
| 36 | Aufhängung |
| 38 | Gelenkglied |
| 40 | Exzenter |
| 42 | Führungsscheibe |
| 44 | Spannringträger |
| 46 | Spannring |
| 48 | Antriebswelle |
| 50 | Rührer |

**Patentansprüche**

1. Vorrichtung zur blasenfreien Begasung von Flüssigkeiten, insbesondere von Kulturmedien zur Vermehrung von Gewebekulturen, mit einem Gasaustausch über eine eingetauchte Membranfläche, die mit Hilfe eines Membrankorbes vorgesehen ist, der pendelnd bewegt wird und einen oder mehrere hydrophobe poröse Polyproylen-Membranhohlfäden aufweist, dadurch gekennzeichnet, dass der Membrankorb zusätzlich mit einem oder mehreren hydrophilen porösen Membranhohlfäden versehen ist, erhältlich durch Hydrophilisieren poröser Polypropylen-Membranhohlfäden durch Benetzen mit einem organischen Lösungsmittel und anschliessendes Benetzen mit Wasser.

2. Vorrichtung nach Anspruch 1, gekennzeichnet durch mehrere hydrophobe poröse Polypropylen-Membranhohlfäden und mehrere hydrophile poröse Polypropylen-Membranhohlfäden, die jeweils getrennt beströmbar sind.

3. Verfahren zur blasenfreien Begasung von Flüssigkeiten, insbesondere von Kulturmedien zur Vermehrung von Gewebekulturen, dadurch gekennzeichnet, dass man das Verfahren mit einer Vorrichtung gemäss Anspruch 1 oder 2 durchführt.

4. Verfahren nach Anspruch 3, dadurch gekennzeichnet, dass man intermittierend über die hydrophilen porösen Polypropylen-Membranhohlfäden flüssiges Medium in den Reaktor einleitet und flüssiges Medium aus dem Reaktor abzieht.

## Claims

1. Apparatus for the bubble-free gassing of liquids, especially of culture media for propagating tissue cultures, with a gas exchange across an immersed membrane sheet provided by means of a membrane cage which is moved in pendulum manner and has one or more hydrophobic porous polypropylene hollow membrane filaments, characterised in that the membrane cage is provided in addition with one or more hydrophilic porous hollow membrane filaments, obtainable by hydrophilisation of porous polypropylene hollow membrane filaments by wetting with an organic solvent and subsequent wetting with water.

2. Apparatus according to claim 1, characterised by several hydrophobic porous polypropylene hollow membrane filaments and several hydrophilic porous polypropylene hollow membrane filaments, each of which can be flowed through separately.

3. Process for the bubble-free gassing of liquids, especially of culture media for propagating tissue cultures, characterised in that the process is carried out with an apparatus according to claim 1 or 2.

4. Process according to claim 3, characterised in that intermittently liquid medium is introduced into the reactor, and liquid medium is removed from the reactor, by way of the hydrophilic porous polypropylene hollow membrane filaments.

## Revendications

1. Dispositif d'apport de gaz, sans bulles, à des liquides, notamment à des milieux de culture permettant la multiplication de cultures de tissus, par échange de gaz par l'intermédiaire d'une surface de membrane immergée, présentée par une cage de membrane qui est déplacée d'une manière oscillante et qui comprend un ou plusieurs fils creux de membrane, poreux et hydrophobes, en polypropylène, caractérisé en ce que la cage de membrane est pourvue en outre d'un ou plusieurs fils creux de membrane, poreux et hydrophiles, susceptible d'être obtenus par hydrophilisation de fils creux de membrane, en polypropylène et poreux, par mouillage au moyen d'un solvant organique, suivi d'un mouillage à l'eau.

2. Dispositif suivant la revendication 1, caractérisé par plusieurs fils creux de membrane poreux et hydrophobes en polypropylène et plusieurs fils creux de membrane poreux et hydrophiles en polypropylène qui sont agencés chacun de façon à pouvoir être séparément traversés par un flux.

3. Procédé d'apport de gaz, sans bulles, à des liquides, notamment à des milieux de culture permettant la multiplication de cultures de tissus, caractérisé en ce qu'il est mis en oeuvre au moyen d'un dispositif suivant la revendication 1 ou 2.

4. Procédé suivant la revendication 3, caractérisé en ce que, d'une manière intermittente, on introduit du milieu liquide dans le réacteur et on extrait du milieu liquide hors du réacteur par l'intermédiaire des fils creux de membrane poreux et hydrophiles en polypropylène.

EP 0 240 560 B1

# FIG.1

## FERMENTATION 1

| | |
|---|---|
| Zellinie | : FIB MONO 6 |
| Medium | : ISCOVE'S DMEM, HAM'S F12 1:1 |
| Zusätze | : 2mM Glutamin, 6,25 µg/l Gentamycin |
| Serum | : 2%(v/v) FCS |
| Reaktor | : Volumen:1,5l, Arbeitsvolumen: 1,2l |
| Begasung | : 2m hydrophober Polypropylenschlauch |
| Medien-wechsel | : 2m hydrophiler Polypropylenschlauch |

7

# FIG.2

## FERMENTATION 2

Zellinie     : FIB MONO 6
Medium       : ISCOVE'S DMEM,HAM'S F12 1:1
Zusätze      : 2mM Glutamin,6,25µg/l Gentamycin
Serum        : 4%(v/v) FCS
Reaktor      : Volumen:1,5l , Arbeitsvolumen:1,2l
Begasung     : 2m hydrophober  Polypropylenschlauch
Medien—
wechsel      : 2m hydrophiler  Polypropylenschlauch

# FIG . 3

FERMENTATION  3

Zellinie     : FIB MONO 6
Medium       : ISCOVE'S DMEM, HAM'S F12 1:1
Zusätze      : 2mM Glutamin, 6,25 µg/l Gentamycin
Serum        : 1% (v/v) FCS
Reaktor      : Volumen:1,5l , Arbeitsvolumen : 1,2l
Begasung     : 2,8m hydrophober Polypropylenschlauch
Medienwechsel      : 1m hydrophiler Polypropylenschlauch

# FIG. 4

**Aufbau des Reaktors**

EP 0 240 560 B1

# FIG . 5

FIG.6